# EUROPÄISCHE PATENTSCHRIFT

(11) **EP 2 311 397 B1**
(45) Veröffentlichungstag und Bekanntmachung des Hinweises auf die Patenterteilung: **30.05.2012**
(21) Anmeldenummer: 09013065.9
(22) Anmeldetag: 15.10.2009
(51) Int. Cl.: A61B 17/88, A61B 17/16, A61C 8/00, B25B 23/14, A61B 19/00, A61C 1/18

(54) **Drehmomentbegrenzer**
Torque limiter
Limiteur de couple

(43) Veröffentlichungstag der Anmeldung: 20.04.2011
(73) Patentinhaber: W+S Solutions GmbH, 78532 Tuttlingen (DE)
(72) Erfinder: Wernz, Ulrich, 78532 Tuttlingen (DE); Siebold, Stephan, 78570 Mühlheim (DE)
(74) Vertreter: Stern, Urs

(56) Entgegenhaltungen:
- EP-A1- 0 873 724
- EP-A1- 1 110 512
- WO-A2-2008/075186
- DE-A1- 4 414 963
- DE-A1-102007 051 263
- DE-U-202006 004 027
- GB-A- 2 191 128
- US-A- 5 366 412
- US-A1- 2007 209 486

## Beschreibung

Die Erfindung betrifft einen Drehmomentbegrenzer nach dem Oberbegriff des Anspruchs 1 und ein Verfahren zum Herstellen eines Drehmomentbegrenzers nach dem Oberbegriff des nebengeordneten Verfahrensanspruchs.

### Stand der Technik

In der Chirurgie müssen regelmäßig Schrauben oder andere Bauteile mit definiertem Drehmoment angezogen werden. Dabei soll in Richtung des Anziehens der Schraube ein maximales Drehmoment nicht überschritten werden, um Schäden an den Bauteilen oder an Knochen zu vermeiden. Andererseits sollte ein Lösen der Schraube auch mit einem höheren Drehmoment möglich sein, um bei einer eventuell sich festgefressenen Schraube die Möglichkeit zu haben, diese aus dem Körper wieder zu entfernen. Aus diesem Grund sind Drehmomentbegrenzer, wie sie in der Chirurgie angewendet werden, grundsätzlich unterschiedlich gegenüber Drehmomentbegrenzern, wie sie allgemein in der Mechanik verwendet werden, da bei letzteren überlicherweise in Gegenrichtung ein Freilauf angeordnet ist.

Aus dem Stand der Technik sind Drehmomentbegrenzer bekannt, die mit einem elastischen Körper arbeiten, welcher ein Drehmoment von einer inneren Welle auf einen außen liegenden Rotor übertragen kann. So betrifft die europäische Patentanmeldung EP 2 085 041 A1 ein entsprechendes chirurgisches Instrument. Nachteilig ist allerdings, dass mit dem elastischen Körper das maximale Drehmoment festgelegt ist. Außerdem kann es durch Materialermüdung der Kunststoff-Materialien des elastischen Körpers zu einer Veränderung der Eigenschaften des Instruments kommen. Dies ist nachteilig, da sich das maximale übertragbare Drehmoment ändern kann.

Aus der EP 1 110 512 A1 ist ein Instrument für die Medizin bekannt, welches zur Drehmomentübertragung einen Rotor und einen Käfig mit in dem Käfig angeordneten Wälzkörpern umfasst, wobei die Wälzkörpern in dem Käfig in radial ausgerichteten Führungen geführt sind und in Ausnehmungen des Rotors in radialer Richtung gedrückt werden, um einen bestimmten Widerstand für eine Drehmomentübertragung mit einem bestimmten maximal übertragbaren Drehmoment herzustellen.

### Offenbarung der Erfindung

Aufgabe der Erfindung ist es, die oben beschriebenen Nachteile des Standes der Technik zu beseitigen oder zumindest zu lindern und einen verbesserten Drehmomentbegrenzer und ein Verfahren zur Herstellung eines verbesserten Drehmomentbegrenzers anzugeben. Insbesondere ist es Aufgabe der Erfindung, einen Drehmomentbegrenzer zu entwickeln, die an verschiedene maximale zu übertragende Drehmomente anpassbar sind und die eine verminderte Veränderung der mechanischen Eigenschaften über die Zeit zeigen.

Ein Drehmomentbegrenzer gemäß dem Hauptanspruch und ein Verfahren zur Herstellung eines entsprechenden Drehmomentbegrenzers entsprechend dem Nebenanspruch werden zur Lösung der Aufgabe vorgesehen. Durch Vorsehen einer geneigten Ebene als Führung für einen Wälzkörper kann durch Auswahl der Winkellage der Neigung ein bestimmter Widerstand gegenüber Verschiebungen des Wälzkörpers entlang der schiefen Ebene erreicht werden. Weiterhin ist durch Auswahl der Anzahl der in dem Käfig aufgenommenen Wälzkörper ein maximal übertragbares Drehmoment auswählbar oder einstellbar. Auf diese Art und Weise kann mit einfachen Mitteln ein maximal übertragbares Drehmoment ausgewählt werden. Es sollte angemerkt werden, dass vorzugsweise gegenüber der Seitenwand, die in einem schiefen Winkel zu einer Umfangsrichtung des Käfigs angeordnet ist, eine zu dieser ersten Seitenwand parallele zweite Seitenwand angeordnet ist, die zusammen mit der ersten Seitenwand die Wälzkörperaufnahme bildet. Dabei wird erreicht, dass einem Verschieben des Wälzkörpers in der Wälzkörperaufnahme je nach Drehrichtung des Käfigs relativ zu dem Rotor ein unterschiedlicher Widerstand entgegengesetzt wird, da das Kraftgleichgewicht an der Auflagestelle des Wälzkörpers auf der Seitenwand je nach Drehrichtung unterschiedlich ist. Die Folge ist, dass in eine Richtung ein maximal übertragbares Drehmoment übertragen werden kann und in eine andere Richtung ein nahezu unbegrenztes oder jedenfalls gegenüber dem maximalen Drehmoment wesentlich erhöhtes Drehmoment übertragen werden kann. Dadurch wird erreicht, dass eine festsitzende Schraube auf jeden Fall gelöst werden kann.

Vorzugsweise ist der Käfig rohrförmig ausgebildet und koaxial zu dem Rotor angeordnet. Die Seitenwand oder die beiden Seitenwände, welche die Wälzkörperaufnahme bilden, sind demnach gegenüber einer radialen Richtung des rohrförmigen Käfigs verkippt, vorzugsweise mindestens um 5°, noch bevorzugter um mindestens 10° oder wenigstens 20°. Der Winkel gegenüber der Radialen beträgt vorzugsweise weniger als 70°, noch bevorzugter weniger als 50° und noch bevorzugter weniger als 35°.

In dem Rotor ist wenigstens eine Vertiefung ausgebildet, die dazu vorgesehen ist, mit dem Wälzkörper zusammenzuwirken. Die Vertiefung bewirkt, dass bei einer Rotation des Rotors relativ zu dem Käfig der Wälzkörper aus dieser Vertiefung heraus angehoben werden muss, wobei er an einer der Seitenwände oder an der führenden der Seitenwände der Wälzkörperaufnahme entlang gleitet. Die Vertiefung ist vorzugsweise als Längsnut in dem Rotor ausgebildet. Vorteilhafterweise sind mehrere Vertiefungen um den Rotor herum angeordnet, bevorzugt mehrere Längsnute, welche in einem lediglich geringen Abstand zueinander oder gar keinem Abstand zueinander angeordnet sind. Durch die oben beschriebene bevorzugte Neigung einer oder beider Seitenwände kommt es in einer Drehrichtung des Käfigs zu nahezu keiner oder zu wenig Reibung und in der Gegenrichtung zu einer nennenswerten Reibung beim Herausdrücken der Wälzkörper. Auf diese Weise wird erreicht, dass unterschiedlich große Drehmomente übertragbar sind.

Die Erfindung umfasst ein Andrückelement zum Andrücken des Wälzkörpers an den Rotor. Das Andrückelement oder die Andrückelemente dienen dazu, einen Wälzkörper in eine Vertiefung zu drücken, sodass bei Herausdrücken eines Wälzkörpers aus einer Vertiefung bei einer Rotation des Rotors gegenüber dem Käfig eine Kraft aufgewendet werden muss. Ausdrücklich umfasst sind bei Nennung einer Einzahl, beispielsweise des Andrückelements oder des Wälzkörpers oder der Vertiefung auch Ausführungsformen, welche eine Mehrzahl der jeweiligen Merkmale aufweisen. Die Erfindung bietet außerdem noch den Vorteil, dass durch Auswahl einer bestimmten Anzahl an Wälzkörpern, Auswahl einer bestimmten Anzahl an Andrückelementen und auch Auswahl bestimmter Andrückelemente mit bestimmten mechanischen Eigenschaften ein maximal übertragbares Drehmoment einfach und zuverlässig festgelegt werden kann. Vorteilhafte Ausführungsformen weisen eine Längsnut als Vertiefung oder Wälzkörper, die als Walze ausgebildet sind, auf.

Vorzugsweise ist das Andrückelement kreisförmig und außerdem vorteilhafterweise koaxial zum Rotor angeordnet. Das Andrückelement kann inner- oder außerhalb des Käfigs angeordnet sein, wobei der Rotor vorzugsweise auf der gegenüber liegenden Seite des Käfigs angeordnet ist. Dies bietet einen möglichst einfachen und kompakten Aufbau. Besondere Vorteile bietet ein innen liegendes Andrückelement, welches innerhalb des Käfigs angeordnet ist. Wird das Andrückelement außerhalb des Käfigs angeordnet und der Rotor innerhalb des Käfigs, kann das Andrückelement ringförmig ausgebildet sein.

Vorzugsweise ist das Andrückelement ein Gummiring oder eine sich in Umfangsrichtung erstreckende Wurmfeder oder eine Federscheibe oder ein Federring oder eine radial angeordnete Spiralfeder. Bevorzugte Ausführungsformen umfassen auch jeweils mehrere oder verschiedene dieser Andrückelemente, beispielsweise um eine Erhöhung des maximal übertragbaren Drehmoments zu erreichen.

Vorzugsweise weist ein als Federscheibe ausgebildetes Andrückelement Federarme auf, welche sich im Wesentlichen in Umfangsrichtung an der Federscheibe erstrecken. Dabei bedeutet "im Wesentlichen" vorzugsweise, dass die Federarme durch eine sich in etwa in Umfangsrichtung, d.h. mit einem Winkel von maximal 20° oder 10° gegenüber der Umfangsrichtung oder genau in Umfangsrichtung, erstreckende Nut von der übrigen Federscheibe getrennt sind. Dies bietet den Vorteil, dass die Federarme in Richtung eines Andrückens des Wälzkörpers an dem Rotor wirken. Die Federarme sind bei typischen Ausführungsformen vorzugsweise spiralförmig angeordnet in einem Winkel zwischen 0° und 20° relativ zu der Umfangsrichtung. Auf diese Weise wird auf geringem Raum der Federarm mit einer ausreichenden Federlänge untergebracht.

Vorteilhafterweise sind der Käfig und die Federscheibe durch eine Verdrehsicherung in einer fixierten Winkellage zueinander gehalten. Auf diese Weise wird sichergestellt, dass die Federarme der Federscheibe an exakt definierten Andrückpunkten auf die Wälzkörper wirken. So wird vermieden, dass bei einer Verdrehung des Käfigs gegenüber der Federscheibe sich der Hebel der Wirkungsrichtung eines Federarms verändert. Auf diese Weise wird sichergestellt, dass die Wälzkörper mit einer definierten Kraft angedrückt werden.

Vorteilhafterweise umfasst die Verdrehsicherung eine Nut, die mit einem Nocken zusammenwirkt. Der Nocken ist vorzugsweise an der Federscheibe angeordnet. Die Nut ist vorzugsweise in dem Käfig angeordnet. Es können auch mehrere Nuten und Nocken über den Umfang der Federscheibe oder des Käfigs verteilt vorgesehen werden. Ebenso ist eine umgekehrte Anordnung möglich, nämlich Nuten in der Federscheibe und Nocken auf dem Käfig. Nuten in dem Käfig weisen den Vorteil auf, dass das Gewicht des Käfigs reduziert wird. Mit den Nuten und Nocken wird erreicht, dass der Federring einfach in einer bestimmten für den Betrieb optimierten Position auf den Käfig aufgeschoben werden kann.

Bei der Erfindung weist der Käfig eine Mehrzahl von Wälzkörperaufnahmen auf, wobei nicht in allen Wälzkörperaufnahmen Wälzkörper vorgesehen sind. Die Anzahl der Wälzkörper ist demnach vorzugsweise kleiner als die Anzahl von Wälzkörperaufnahmen. Auf diese Weise wird eine Verminderung des maximal übertragbaren Drehmoments auf ein gewünschtes Maß erreicht.

Ein weiterer Aspekt der Erfindung betrifft ein Verfahren zum Herstellen eines Drehmomentbegrenzers in einer der oben beschriebenen bevorzugten Ausführungsformen, wobei ein maximal durch den Drehmomentbegrenzer in einer Drehrichtung übertragbares Drehmoment eingestellt wird durch eine Auswahl einer bestimmten Anzahl von Wälzkörpern, die in die Wälzkörperaufnahme eingesetzt werden und eine Auswahl des Typs und der Anzahl von Andrückelementen, die in den Drehmomentbegrenzer eingebaut werden. Dabei ist mit Typ von Andrückelementen gemeint, dass unterschiedlich ausgebildete Federscheiben oder Gummiringe oder Wurmfedern verwendet werden können. So können dickere und dünnere Federscheiben vorgesehen sein, wobei die dünneren Federscheiben einen geringeren Anpressdruck erzeugen als die dickeren Federscheiben. Ebenso können die Federarme verschiedener Federscheiben unterschiedlich ausgestaltet sein, um unterschiedliche Andrückkräfte zu erreichen. Bei bevorzugten Herstellverfahren in Abhängigkeit eines maximal in einer Drehrichtung zu übertragenden Drehmoments wird festgelegt, welche Anzahl von Wälzkörpern eingesetzt werden. Ebenso wird bevorzugt in Abhängigkeit des geforderten maximal übertragbaren Drehmoments eine Auswahl des Typs oder der Anzahl von Andrückelementen vorzunehmen.

Der Drehmomentbegrenzer bietet den besonderen Vorteil, dass Anwenderseitig leicht durch Auswahl bestimmter kategorisierter Bauelemente, wie Wälzkörper oder Andrückelemente, ein maximal übertragbares Drehmoment eingestellt werden kann. Weiterhin bietet ein erfindungsgemäßer Drehmomentbegrenzer den Vorteil, dass er leicht vollständig sterilisierbar ist. Ein bevorzugter Drehmomentbegrenzer umfasst zumindest zwei Wälzkörper und einen Käfig mit mindestens zwei Wälzkörperaufnahmen. Der Anwender kann durch Auswahl der Teile wie Anzahl der Wälzkörper oder Andrückelemente ein maximal übertragbares Drehmoment auswählen. Ebenso können vorzugsweise drei oder mehr Wälzkörperaufnahmen und Wälzkörper Bestandteil des Drehmomentbegrenzers sein. Weiterhin wird bevorzugt, dass der Drehmomentbegrenzer zumindest zwei Andrückelemente umfasst. Durch Auswahl der Anzahl der Andrückelemente, welche später in dem Drehmomentbegrenzer verwendet werden, kann ein maximal übertragbares Drehmoment eingestellt werden. Weiterhin wird bevorzugt, dass der Drehmomentbegrenzer zumindest zwei unterschiedliche Andrückelemente umfasst. Auf diese Weise können unterschiedliche maximal übertragbare Drehmomente ausgewählt werden.

### Kurze Beschreibung der Zeichnungen

Nachfolgend wird ein bevorzugtes Ausführungsbeispiel der Erfindung anhand der beiliegenden Zeichnungen beschrieben, wobei die beiliegenden Zeichnungen zeigen:
Fig. 1 zeigt eine Explosionsdarstellung eines erfindungsgemäßen Drehmomentbegrenzers in einer schematischen perspektivischen Ansicht;
Fig. 2 zeigt einen Teil des Drehmomentbegrenzers der Fig. 1 in zusammengebauter Form in einer geschnittenen schematischen Ansicht, wobei der Schnitt entlang der Längsachse des erfindungsgemäßen Drehmomentbegrenzers gewählt ist;
Fig. 3 zeigt einen Querschnitt durch den zusammengesetzten Drehmomentbegrenzer mit Bauteilen, welche in der Fig. 1 gezeigt sind; und
Fig. 4 zeigt einen Querschnitt durch ein anderes Ausführungsbeispiel eines erfindungsgemäßen Drehmomentbegrenzers.

### Beschreibung eine bevorzugter Ausführungsbeispiele

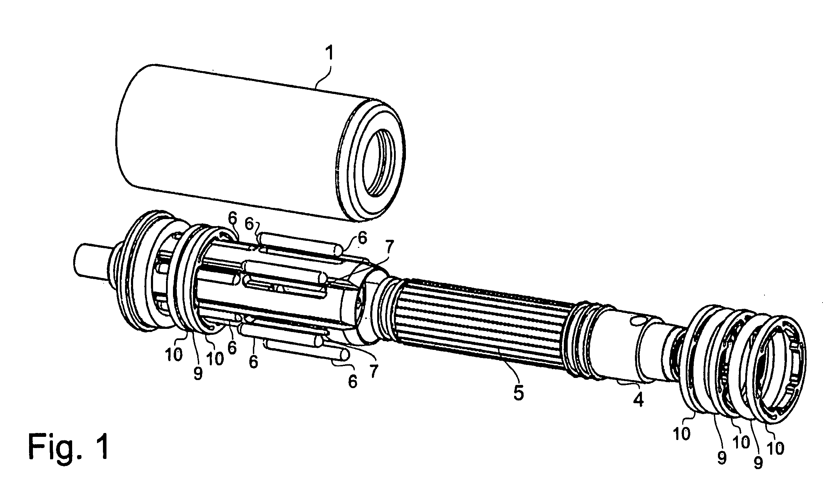

Die Fig. 1 zeigt in schematischer Explosionsdarstellung perspektivisch einen erfindungsgemäßen Drehmomentbegrenzer bzw. einen erfindungsgemäßen Bausatz für einen Drehmomentbegrenzer. Der Drehmomentbegrenzer verfügt über eine äußere Schutzhülle 1, eine Abdeckkappe sowie mehrere Lager. Die Abtriebswelle des Drehmomentbegrenzers wird integral durch einen innen liegenden Rotor 4 gebildet, auf dessen Außenseite Längsnuten als Vertiefungen 5 angeordnet sind. Die Längsnuten als Vertiefungen 5 sind dazu vorgesehen, mit Wälzkörpern 6 zusammenzuwirken, wobei der Innenradius der Längsnuten dem Außenradius der Wälzkörper 6 entspricht. Als Wälzkörper 6 sind an den Enden abgerundete Walzen vorgesehen. Bei anderen typischen Ausführungsformen sind Walzen vorgesehen, die scharfkantige Enden aufweisen. Die Wälzkörper 6 sind in Wälzkörperaufnahmen 7 eines Käfigs 8 aufgenommen. Der Käfig 8 bildet gleichzeitig eine Antriebswelle des Drehmomentbegrenzers. Selbstverständlich können Antrieb und Abtrieb des Drehmomentbegrenzers ebenso vertauscht sein.

Die Wälzkörper 6 werden durch Andrückelemente in den Wälzkörperaufnahmen 7 gehalten. Als Andrückelemente werden Gummiringe 9 und Federscheiben 10 verwendet. Es sollte angemerkt werden, dass in dem dargestellten Ausführungsbeispiel die Gummiringe 9 kaum oder keine Wirkung auf das übertragbare Drehmoment haben, da die Federscheiben 10 wesentlich steifer sind. Allerdings erleichtern die Gummiringe 9 auch die Montage der Wälzkörper 6 in dem Käfig 8, da die Gummiringe 9 die Wälzkörper 6 gegen ein Herausfallen sichern. Die Andrückelemente bringen eine radiale Kraft auf die Wälzkörper 6 auf und drücken die Wälzkörper 6 somit in die Wälzkörperaufnahmen 7 und gegen den Rotor 4. Dabei sind in einer Position mit minimierter Energie der Andrückelemente die Wälzkörper 6 in jeweils einer Vertiefung 5, d.h. einer Längsnut, des Rotors 4 angeordnet.

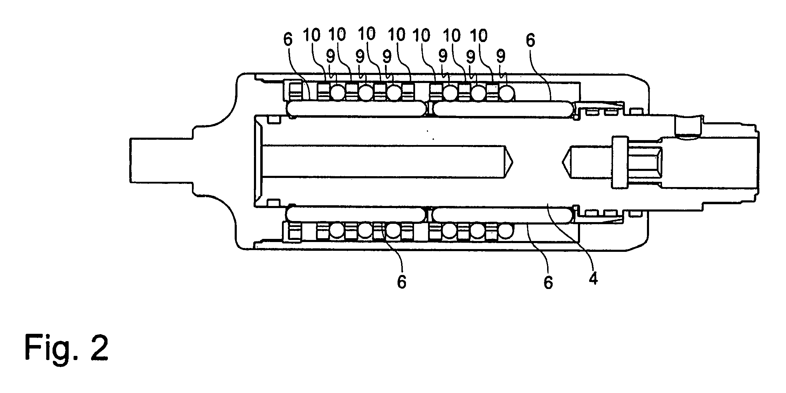

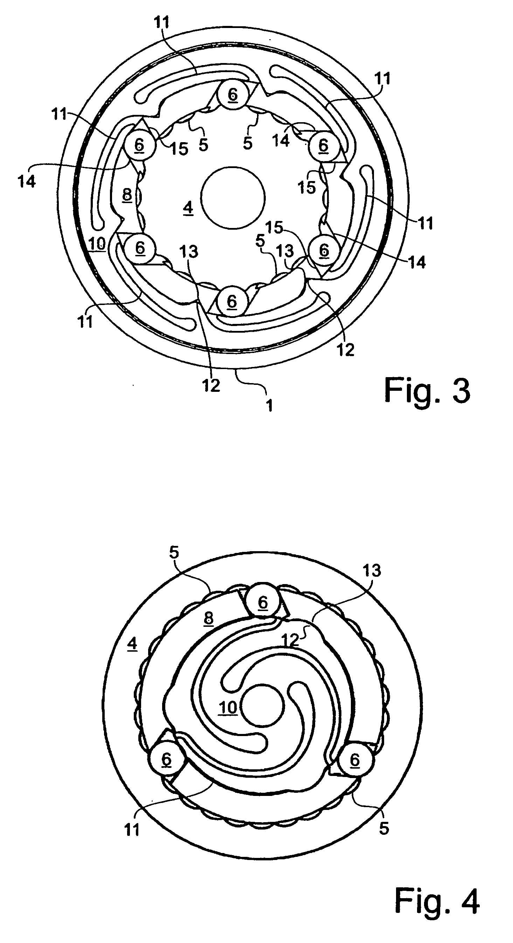

Bei einer relativen Verdrehung des Käfigs 8 gegenüber dem Rotor 4 müssen daher die Wälzkörper 6 gegen die Kraft der Andrückelemente, d.h. der Gummiringe 9 und der Federscheiben 10, gedrückt werden, sodass die Wälzkörper 6 von einer Vertiefung 5 in eine nächste Vertiefung 5 des Rotors 4 gelangen können. Dabei spielt auch eine Rolle, dass die Seitenwände der Wälzkörperaufnahmen 7 nicht exakt radial in dem Käfig 8 ausgebildet sind, sondern in einem schiefen Winkel zu der Oberfläche des Käfigs 8 angeordnet sind. Dadurch wird ein Anheben der Wälzkörper 6 in den Wälzkörperaufnahmen 7 bei einem Verdrehen des Rotors 4 gegenüber dem Käfig 8 in einer relativen Drehrichtung vereinfacht gegenüber der entgegengesetzten Drehrichtung. Dies bedeutet, dass in einer Drehrichtung ein geringeres Drehmoment aufgebracht werden muss, um den Rotor 4 gegenüber dem Käfig 8 zu verdrehen als in der entgegengesetzten Drehrichtung. Die schräg angeordneten Seitenwände der Wälzkörperaufnahmen 7 werden im Zusammenhang mit den Figuren 2 und 3 erläutert.

Es sollte angemerkt werden, dass in den Figuren 1 bis 3 für gleiche oder für ähnliche Teile gleiche Bezugszeichen verwendet werden, wobei außerdem nicht in jeder Zeichnung jedes Teil noch einmal mit einem Bezugszeichen versehen ist, um die Übersichtlichkeit zu erhöhen.

Die Fig. 2 zeigt einen Längsschnitt durch einen zusammengesetzten Drehmomentbegrenzer. Zur Erläuterung wird auf die Beschreibung zur Fig. 1 verwiesen. Es sollte angemerkt werden, dass bei einem Zusammenbau des Drehmomentbegrenzers nicht alle Wälzkörperaufnahmen 7 mit Wälzkörpern 6 besetzt werden müssen. Ebenso ist in der Fig. 2 zu erkennen, dass nicht alle Wälzkörper 6 jeweils durch Andrückelemente gegen den Rotor 4 gedrückt werden. Es ist möglich, noch weitere Andrückelemente vorzusehen, um ein durch den Drehmomentbegrenzer maximal übertragbares Drehmoment zu erhöhen. Ebenso ist es möglich, einige der Gummiringe 9 oder der Federscheiben 10 wegzulassen, um ein maximal übertragbares Drehmoment zu reduzieren.

In der Fig. 3 ist ein Querschnitt durch den erfindungsgemäßen Drehmomentbegrenzer gezeigt, wobei der Schnitt auf Höhe einer der Federscheiben 10 (siehe Fig. 2) angeordnet ist. Zur Erläuterung wird wiederum auf die Figuren 1 und 2 und die dazu gehörigen Figurenbeschreibungen verwiesen. In der Fig. 3 sind außerdem Federarme 11 der Federscheibe 10 gezeigt, welche als Feder die Wälzkörper 6 gegen den Rotor 4 drücken.

Außerdem sind an den Federscheiben 10 Nocken 12 angeordnet, welche in Nuten 13 des Käfigs 8 eingreifen. Die Nuten 13 und die Nocken 12 dienen dazu, eine Verdrehsicherung zwischen der Federscheibe 10 und dem Käfig 8 herzustellen. Auf diese Weise wird sichergestellt, dass der Kontakt zwischen den Wälzkörpern 6 und den Federarmen 11 an bestimmten Punkten oder bestimmten Bereichen der Federarme 11 hergestellt wird. Auf diese Weise wird erreicht, dass die Wälzkörper 6 mit einer definierten Kraft gegen den Rotor 4 gedrückt werden.

Außerdem ist in der Fig. 3 gezeigt, dass die Wälzkörperaufnahmen 7 über eine erste schräge Seitenwand 14 und eine zweite schräge Seitenwand 15 verfügen. Die erste schräge Seitenwand 14 und die zweite schräge Seitenwand 15 sind dabei parallel zueinander ausgerichtet und außerdem jeweils in einem Winkel von etwa 30° gegenüber einer radialen Richtung des Käfigs 8 geneigt. Dies bewirkt, dass ein Anheben der Wälzkörper 6 in radialer Richtung bei einer relativen Verdrehung des Rotors 4 gegenüber dem Käfig 8 in eine erste Drehrichtung einfacher ist als in eine entgegengesetzte zweite Drehrichtung. So ist in der Fig. 3 eine Verdrehung des Rotors 4 im Uhrzeigersinn bzw. eine Verdrehung des Käfigs 8 gegen den Uhrzeigersinn mit einem geringeren Drehmomentaufwand verbunden als eine entgegengesetzte relative Verdrehung. Der Grund hierfür ist, dass bei einem Anheben der Wälzkörper 6 in den Wälzkörperaufnahmen 7 durch den Rotor 4 die Erhebungen zwischen den Vertiefungen 5 des Rotors 4 auch bewirken, dass die Wälzkörper 6 in der relativen Drehrichtung des Rotors 4 angetrieben werden. Dabei kommt es darauf an, ob den Wälzkörpern 6 in dieser Bewegungsrichtung eine schräg "nach oben" geneigte Seitenwand entgegensteht oder eine schräg "nach unten" geneigte, d.h. entgegen der Bewegungsrichtung geneigte.

Andere typische Ausführungsformen weisen zwei unterschiedlich geneigte Seitenwände auf. Ebenfalls möglich ist die Anordnung der Seitenwande in einer symmetrischen V-Form, so dass in beiden Drehrichtungen das gleiche Drehmoment übertragbar ist.

Mit der gezeigten Anordnung wird vorteilhafterweise erreicht, dass auch bei längerer Gebrauchsdauer gleichbleibende oder nahezu gleichbleibende mechanische Eigenschaften des Drehmomentbegrenzers erreicht werden und außerdem ein Herausdrehen auch mit einem wesentlich höheren Drehmoment möglich ist als ein Hineindrehen, welches lediglich mit dem durch den Drehmomentbegrenzer maximalen Drehmoment möglich ist.

In der Fig. 4 ist in einer schematischen Skizze eine weitere Ausführungsform eines erfindungsgemäßen Drehmomentbegrenzers gezeigt. Bei der Skizze der Fig. 4 werden für gleiche oder ähnliche Teile gleiche Bezugszeichen verwendet.

Der Drehmomentbegrenzer, welcher in der Fig. 4 dargestellt ist, weist einen innenliegenden Käfig 8 und einen aussenliegenden Rotor 4 auf. Eine innenliegende Federscheibe 10 mit spiralförmig angeordneten Federarmen 11 drückt Wälzkörper 6 nach aussen in Längsnuten des Rotors 4. Die Federarme 11 sind spiralförmig an der Federscheibe 10 ausgebildet. An den Federarmen 11 ausgebildete Nocken 12 und in dem Käfig 8 angeordnete Nuten 13 bilden eine Verdrehsicherung. Die dargestellte Ausführungsform mit dem innen liegenden Rotor 4 weisten den Vorteil einer kompakten Bauform auf. Der übrige Aufbau und die übrigen Alternativen, beispielsweise für Andrückelemente können entsprechend dem vorherig beschriebenen Ausführungsbeispiel vorgesehen werden.

## Patentansprüche

1. Drehmomentbegrenzer, insbesondere für ein chirurgisches Instrument, mit
- einem Rotor (4) und
- einem koaxial zu dem Rotor (4) angeordneten Käfig (8), der zumindest eine Wälzkörperaufnahme (7) mit einem darin aufgenommen Wälzkörper (6) aufweist,
- wobei die Wälzkörperaufnahme (7) zur Führung für den Wälzkörper (6) zumindest eine Seitenwand (15) aufweist,
**dadurch gekennzeichnet, dass**
- die Seitenwand (15) zur Führung für den Wälzkörper (6) in einem schiefen Winkel zu einer Umfangsrichtung des Käfigs (8) und verkippt gegenüber einer radialen Richtung des Käfigs (8) angeordnet ist,
- der Rotor (4) wenigstens eine Vertiefung (5) umfasst, die dazu vorgesehen ist, mit dem Wälzkörper (6) zusammenzuwirken, und
**gekennzeichnet durch**
- ein Andrückelement zum Andrücken des Wälzkörpers (6) an den Rotor (4).

2. Drehmomentbegrenzer nach Anspruch 1, **dadurch gekennzeichnet, dass** der Wälzkörper (6) als Walze ausgeführt ist und/oder die Vertiefung (5) als Längsnut in dem Rotor (4) ausgebildet ist.

3. Drehmomentbegrenzer nach Anspruch 2, **dadurch gekennzeichnet, dass** das Andrückelement kreisförmig oder ringförmig ist.

4. Drehmomentbegrenzer nach Anspruch 2 oder 3, **dadurch gekennzeichnet, dass** als Andrückelement ein Gummiring (9) und/oder eine Wurmfeder und/oder eine Federscheibe (10) und/oder eine Spiralfeder vorgesehen sind.

5. Drehmomentbegrenzer nach einem der vorhergehenden Ansprüche, **dadurch gekennzeichnet, dass** das Andrückelement eine Federscheibe (10) ist, die sich im Wesentlichen in Umfangsrichtung erstreckende Federarme (11) aufweist.

6. Drehmomentbegrenzer nach Anspruch 5, **dadurch gekennzeichnet, dass** der Käfig (8) und die Federscheibe (10) durch eine Verdrehsicherung in einer fixierten Winkellage zueinander gehalten sind.

7. Drehmomentbegrenzer nach Anspruch 6, **dadurch gekennzeichnet, dass** die Verdrehsicherung eine Nut (13), die mit einem Nocken (12) zusammenwirkt, umfasst.

8. Drehmomentbegrenzer nach einem der vorhergehenden Ansprüche, **dadurch gekennzeichnet, dass** eine der ersten Seitenwand (15) gegenüberliegende zweite Seitenwand (14) der Wälzkörperaufnahme (7) im Wesentlichen parallel zu der ersten Seitenwand (15) ausgerichtet ist.

9. Drehmomentbegrenzer nach einem der vorhergehenden Ansprüche, **dadurch gekennzeichnet, dass** eine Mehrzahl von Wälzkörperaufnahmen (7) und eine Mehrzahl von Wälzkörpern (6) in dem Käfig (8) vorgesehen sind, wobei die Anzahl der Wälzkörper (6) kleiner ist als die Anzahl von Wälzkörperaufnahmen (7).

10. Verfahren zum Herstellen eines Drehmomentbegrenzers nach einem der Ansprüche 1 bis 9, mit den Schritten:
- Anordnen eines Käfigs (8) koaxial zu einem Rotor (4), wobei der Käfig (8) eine Mehrzahl von Wälzkörperaufnahmen (7) mit jeweils zumindest einer Seitenwand (15) zur Führung von Wälzkörpern (6) aufweist, und wobei die Seitenwand (15) jeweils in einem schiefen Winkel zu einer Umfangsrichtung des Käfigs (8) und verkippt gegenüber einer radialen Richtung des Käfigs (8) angeordnet ist, und wobei der Rotor eine Mehrzahl von Vertiefungen umfasst, die dazu vorgesehen sind, mit den Wälzkörpern zusammenzuwirken,
- Bestimmen einer Anzahl von Wälzkörpern (6) und Auswahl des Typs und Auswahl der Anzahl von Andrückelementen zum Andrücken der Wälzkörper (6) an den Rotor (4) in Abhängigkeit eines in einer Drehrichtung übertragbaren Drehmoments und
- Einsetzen der bestimmten Anzahl von Wälzkörpern (6) in die Wälzkörperaufnahmen (7) und Einbau der ausgewählten Andrückelemente zum Andrücken der Wälzkörper (6) an den Rotor (4).

## Claims

1. Torque limiter, in particular for a surgical instrument, comprising
- a rotor (4) and
- a cage (8) which is disposed coaxially with respect to the rotor (4) and has at least one rolling element holder (7) with a rolling element (6) held therein,
- wherein the rolling element holder (7) has at least one side wall (15) for guiding the rolling element (6),
**characterised in that**
- the side element (15) for guiding the rolling element (6) is disposed at an oblique angle to a circumferential direction of the cage (8) and tilted relative to a radial direction of the cage (8),
- the rotor (4) comprises a depression (5) which is provided in order to co-operate with the rolling element (6), and
**characterised by**
- a pressing element for pressing the rolling element (6) onto the rotor (4).

2. Torque limiter as claimed in Claim 1, **characterised in that** the rolling element (6) is configured as a roller and/or the depression (5) is formed as a longitudinal groove in the rotor (4).

3. Torque limiter as claimed in Claim 2, **characterised in that** the pressing element is circular or annular.

4. Torque limiter as claimed in Claim 2 or Claim 3, **characterised in that** a rubber ring (9), a worm spring and/or a spring washer (10), and/or a spiral spring are provided as the pressing element.

5. Torque limiter as claimed in any one of the preceding claims, **characterised in that** the pressing element is a spring washer (10) which has spring arms (11) extending substantially in the circumferential direction.

6. Torque limiter as claimed in Claim 5, **characterised in that** the cage (8) and the spring washer (10) are held in a fixed angular position relative to one another by an anti-torsion means.

7. Torque limiter as claimed in Claim 8, **characterised in that** the anti-torsion means comprises a groove (13) which co-operates with a cam (12).

8. Torque limiter as claimed in any one of the preceding claims, **characterised in that** a second side wall (14) of the rolling element holder (7) opposite the first side wall (15) is aligned substantially parallel to the first side wall (15).

9. Torque limiter as claimed in any one of the preceding claims, **characterised in that** a plurality of rolling element holders (7) and a plurality of rolling elements (6) are provided in the cage (8), wherein the number of rolling elements (6) is less than the number of rolling element holders (7).

10. Method for producing a torque limiter as claimed in any one of Claims 1 to 9, with the steps:
- disposing a cage (8) coaxially relative to a rotor (4), wherein the cage (8) has a plurality of rolling element holders (7) each having at least one side wall (15) for guiding rolling elements (6), and wherein the side wall (15) is in each case disposed at an oblique angle to a circumferential direction of the cage (8) and tilted relative to a radial direction of the cage (8),
- and wherein the rotor (4) comprises a plurality of depressions (5) which are provided in order to co-operate with the rolling element (6),
- determining a number of rolling elements (6) and selecting the type and selecting the number of pressing elements for pressing the rolling elements (6) onto the rotor (4) as a function of a torque which can be transmitted in one direction of rotation, and
- inserting the determined number of rolling elements (6) into the rolling element holders (7) and installing the selecting pressing elements for pressing the rolling elements (6) onto the rotor (4).

## Revendications

1. Limiteur de couple, en particulier pour un instrument chirurgical, avec
- un rotor (4), et
- une cage (8), disposée coaxiale au rotor (4), qui présente au moins un dispositif de réception de corps rotatif (7) avec un corps rotatif (6) y reçu,
- le dispositif de réception de corps rotatif (7) présentant, pour le guidage du corps rotatif (6), au moins une paroi latérale (15),
**caractérisé par le fait que**
- la paroi latérale (15) est, pour le guidage du corps rotatif (6), disposée selon un angle oblique par rapport à une direction périphérique de la cage (8) et basculée par rapport à une direction radiale de la cage (8),
- le rotor (4) comporte au moins un creux (5) qui est prévu pour coopérer avec le corps rotatif (6), et
**caractérisé par**
un élément de pression destiné à presser le corps rotatif (6) contre le rotor (4).

2. Limiteur de couple selon la revendication 1, **caractérisé par le fait que** le corps rotatif (6) est réalisé sous forme de rouleau et/ou que l'évidement (5) est réalisé sous forme de rainure longitudinale dans le rotor (4).

3. Limiteur de couple selon la revendication 2, **caractérisé par le fait que** l'élément de pression est en forme de cercle ou en forme d'anneau.

4. Limiteur de couple selon la revendication 2 ou 3, **caractérisé par le fait qu'**il est prévu, comme élément de pression, une bague en caoutchouc (9) et/ou un ressort en forme de vis sans fin et/ou une rondelle à ressort (10) et/ou un ressort hélicoïdal.

5. Limiteur de couple selon l'une des revendications précédentes, **caractérisé par le fait que** l'élément de pression est une rondelle à ressort (10) qui présente des bras de ressort (11) s'étendant sensiblement dans le sens du pourtour.

6. Limiteur de couple selon la revendication 5, **caractérisé par le fait que** la cage (8) et la rondelle à ressort (10) sont maintenues par un dispositif anti-rotation dans une position angulaire fixe l'une par rapport à l'autre.

7. Limiteur de couple selon la revendication 6, **caractérisé par le fait que** le dispositif anti-rotation comporte une rainure (13) qui coopère avec une came (12).

8. Limiteur de couple selon l'une des revendications précédentes, **caractérisé par le fait qu'**une deuxième paroi latérale (14), opposée à la première paroi latérale (15), du dispositif de réception de corps rotatif (7) est orientée sensiblement parallèle à la première paroi latérale (15).

9. Limiteur de couple selon l'une des revendications précédentes, **caractérisé par le fait qu'**une pluralité de dispositifs de réception de corps rotatif (7) et une pluralité de corps rotatifs (6) sont prévus dans la cage (8), le nombre de corps rotatifs (6) étant inférieur au nombre de dispositifs de réception de corps rotatif (7).

10. Procédé de fabrication d'un limiteur de couple selon l'une des revendications 1 à 9, aux étapes consistant à:
- disposer une cage (8) coaxialement à un rotor (4), la cage (8) présentant une pluralité de dispositifs de réception de corps rotatif (7), chacun avec au moins une paroi latérale (15) destinée à guider des corps rotatifs (6), et la paroi latérale (15) étant chaque fois disposée selon un angle oblique par rapport à une direction périphérique de la cage (8) et basculée par rapport à une direction radiale de la cage (8), et le rotor comportant une pluralité d'évidements qui sont prévus pour coopérer avec les corps rotatifs,
- déterminer un nombre de corps rotatifs (6) et sélectionner le type et sélectionner le nombre d'éléments de pression des corps rotatifs (6) contre le rotor (4) en fonction d'un couple de rotation transmis dans une direction de rotation, et
- utiliser le nombre déterminé de corps rotatifs (6) dans les dispositifs de réception de corps rotatif (7) et incorporation des éléments de pression sélectionnés pour presser les corps rotatifs (6) contre le rotor (4).
